# EUROPEAN PATENT APPLICATION

(11) **EP 3 581 546 A1**
(43) Date of publication of application: **18.12.2019**
(21) Application number: 18752002.8
(22) Date of filing: 07.02.2018
(51) Int. Cl.: C02F 11/02

(54) **SCALABLE BIODIGESTER PLANT FOR GENERATING BIOGAS FROM LIVESTOCK PURINES**

(30) Priority: 07.02.2017 ES 201700078 U
(71) Applicant: Mas Rodriguez, Mario José, 35018 Las Palmas De Gran Canaria (ES)
(72) Inventor: Mas Rodriguez, Mario José, 35018 Las Palmas De Gran Canaria (ES)
(74) Representative: Del Valle Valiente, Sonia
(86) International application number: PCT/ES2018/070088
(87) International publication number: WO 2018/146363

(57) **Abstract**

The invention relates to a scalable biodigester plant for generating biogas from livestock liquid manure, comprising a concentration tank (2) with a stirrer (3) associated with a pretreatment tank (5) and treatment tanks (6) by means of a liquid manure pipe (4), with a heating pipe (7) communicating said tanks (5, 6) with a heater/generator (8), a biogas pipe (9) communicating the tanks (5, 6) with the heater/generator (8), and a discharge pipe (10) communicating the tanks (5, 6) with the solid/liquid separator (11), the tanks (5, 6) being movable and consisting of 20' or 40' maritime transport containers open in the upper part, and an expandable rubber dome (12) that seals said upper part allowing only the passage of the biogas pipe (9), the containers being covered internally with a thermally insulating layer (13) that is, in turn, covered with a reinforcement layer (14).

## Description

### OBJECT OF THE INVENTION

As expressed by the title of the present specification, the invention relates to a scalable biodigester plant for generating biogas from livestock liquid manure which adds to the function for which it is intended certain advantages and novelty features described in detail below which constitute an improvement to the current state of the art.

More particularly, the object of the invention focuses on a plant of the type intended to be installed on livestock farms to utilize livestock liquid manure or other agro-industry waste for producing gas by means of a standing and heating process through a series of tanks into which the liquid manure is successively transferred, which plant contemplates in an advantageous and innovative manner the use of standard maritime transport containers suitably prepared for said function, in lieu of conventional tanks made up of fixed concrete pits, allowing, among other advantages, the mobility thereof and the scalability of the plant in a quick and simple manner, without any need for any civil engineering work or major investments.

### FIELD OF APPLICATION OF THE INVENTION

The field of application of the present invention is in the sector of the industry dedicated to the installation of recycling and waste treatment plants, particularly focusing on the field of liquid manure treatment for obtaining biogas.

### BACKGROUND OF THE INVENTION

As a reference to the current state of the art, it should be pointed out that liquid manure biodigestion plants and facilities like the one referred to herein, as well as the method that is followed for obtaining biogas from same, are known.

However, at least on the part of the applicant, there is no known existence of a plant of said type presenting technical and constitutive features that are identical or similar to those specifically proposed herein, as claimed, and which have the primary objective of providing means which allow saving in costs and complexity in facilities of this type, as well as allowing the scalability of such plants given that those existing at present usually feature fixed tanks made from concrete structures, usually pits, which requires huge investments which hardly allow modifying the work capacity based on eventual variations in usage needs.

### DESCRIPTION OF THE INVENTION

The scalable biodigester plant for generating biogas from livestock liquid manure proposed by the invention is thus configured as a remarkable novelty within its field of application all the objectives indicated above are unquestionably met as a result of its implementation, with the characterizing details being described in the final claims attached to the present description.

Specifically and as mentioned above, the invention proposes a plant intended to be installed in livestock farms to utilize the liquid manure of its animals for producing biogas by means of a biodigestion process, which plant contemplates in an advantageous and innovative manner the use of maritime transport containers as tanks prepared for carrying out said process, advantageously allowing for its mobility and easy scalability of the plant, among other advantages.

More specifically, the process starts in the livestock shed the concrete slab of which allows collecting liquid manure by means of a system of scrapers which move it and dump it into the liquid manure pit.

Once the liquid manure is collected in the mentioned pit, it goes to a concentration tank with a stirrer to homogenize the mixture formed by said liquid manure.

Once homogenized, the liquid manure is pumped to a first pretreatment tank, the first tank constituting the plurality the plant may feature, where the liquid manure is subject to heating of 70°C for two hours.

One of the advantages of the plant object of the invention is that it can be operated so that said plurality of tanks are filled with the liquid manure in series or in parallel. This means that it can be loaded by means of completely filling the first tank and, by overflow, filling the following tank, that is, in series. This loading system is particularly useful when the generation of liquid manure is very small in relation to the capacity of the tank. If the filling and hold period in each container is not enough to obtain a stable biodigestion process, the system could be managed in parallel, distributing the load into two or more tanks, increasing the hold time in each.

Thus, once pretreatment at 70ºC is overcome, the liquid manure begins to pass through, due to overflow, a pipe provided for that purpose, whereby communicating all the tanks of the plant, into the following tank until filling it, as occurs with the third tank, and so on and so forth until filling all the tanks the plant features, with the particularity that in the plant proposed by the invention, said tanks are standard 20' (6 meters long and with a capacity of about 31.1 m3) or 40' (12 meters long and with a capacity of 62 m3) maritime transport containers, in any case of the type referred to as "open top", the upper part of which is open, which allows hermetically covering them with a special expandable EPDM rubber (ethylene propylene diene monomer rubber).

Furthermore, each container is internally lined with insulating material, preferably with 30 mm high-density polyurethane, covered in turn with a layer of polyester and glass fiber, to achieve suitable water and thermal insulation. The container will be sealed at its upper part with the mentioned EPDM rubber such that, through an upper pipe, it allows capturing the biogas generated.

This system allows transferring the largest bacterial load from one tank to another, aiding in the biodigestion process. With a number of 150 milk cows, for example, 11 m3 of liquid manure will be generated per day, so it will take three days to fill each 20' container. The 150 cows will produce 4.5 tons of liquid manure daily, which will generate 93 m3 of biogas per day.

Each tank must have a small pump or other hydraulic, mechanical or pneumatic system for circulating the liquid manure, which is activated periodically, for example, for 10 to 15 minutes every hour, thus preventing crust formation.

Furthermore, each tank features the following pipes:
- A heating pipe, which takes hot water from a heater/combined cycle generator provided for that purpose, to keep the temperature around the 37°C, which is the ideal temperature for biodigestion. Said heating pipe is located in the low part of the container, since it thus performs better because hot liquid manure tends to rise to the top. For a higher yield, the heater/generator must be located very close to the first tank or pre-heating tank, to offer the highest temperature required in it and prevent heat losses.
- A liquid manure inlet pipe, in the upper part of the tank, through which the tank is filled with the liquid manure.

- A liquid manure outlet pipe, in the upper part opposite the inlet pipe, and connected to the following tank.
- A biodigester discharge and complete cleaning pipe, located in the lower part of the tank, which will be used to discharge and clean the container if needed.
- A biogas outlet pipe, placed in the upper part of the tank, in the rubber cover.
- And, optionally, taps for taking samples.

Each pipe has its corresponding opening and closing valve.

Furthermore, each tank also incorporates inlets and outlets for a probe, for measuring temperature.

During the liquid manure hold cycle in the tanks, 25 to 30 days, the liquid manure circulates through the tanks, generating the biogas that will be used to move a generator (or microturbine) with the boiler integrated. The heat output is used, together with the boiler, to inject heat into the entire system. In any case, this heater/generator is also located in a 20' maritime transport container, which is located right next to the container constituting the pretreatment tank.

Finally, a solid/liquid separator is incorporated at the outlet of the final tank in the series to extract the largest possible amount of solids. Said extracted solids are composted to be sold as dry manure, and the liquid extracts as fertilizer, whereby utilizing everything produced in the plant.

The digestate obtained is stored in recipients with a capacity of 1000 liters or more for distribution among interested farmers.

Before the biogas reaches the generator, it must be subjected to filtration to reduce the presence of hydrogen sulfide and to reduce moisture.

An automatic burner connected to a solar panel can be installed to burn off the excess biogas that is not consumed, which prevents methane from reaching the atmosphere.

Finally, it should be mentioned that although the plant is primarily intended for using livestock liquid manure, it must be understood that it could also be utilized for other agro-industry waste, sludge for example.

Therefore, the main advantages provided by the plant object of the invention are the following:
- It features tanks consisting of maritime transport containers that are thermally insulated and reinforced with polyester and glass fiber to generate the biogas. The polyester plus the layer of glass fiber internally provided add important qualities such as durability, thermal insulation, resistance to abrasive products, etc.

- It is scalable, that is, its structure can be increased or modified without making enormous investments and using the existing system. When the farm wants to hold a larger number of animals, and therefore requires a liquid manure biodigester plant with a higher capacity, it will simply add more containers to the series connected for carrying out the described biodigestion process. In most existing systems, major investments must be made in new concrete pits or tanks. The only limitation is determined by the generator, which has a maximum biogas generation and consumption capacity. However, there are scalable generators which may help increase generation without major investments.
- Recovery of the system. Since the plant is divided into multiple containers, and since the containers are movable, any element affecting the system, for example, the sudden death of bacteria in one of the tanks (if detected in time) or physical damage in the tank, the container that has been affected can be isolated from the rest of the tanks of the plant. It is isolated simply by means of closing the valves, repaired, and incorporated back into biogas production once repaired.
- Ease of installation and operation. Construction permits and licenses are considerably reduced. Its installation is much simpler since all the elements travel and operate in transportable containers that are pre-installed, which facilitates transport and start-up.
- It can be disassembled. This distinguishes it from any other plant or installation. It can be integrally moved and re-installed in another site. This is very useful when the manager of the farm is not the owner of the property.
- It allows the possibility of operating continuously or discontinuously. When it is operated continuously, all the tanks of the plant are simultaneously filled by overflow. This system has the advantage that it is easier to put the second tank into operation as it has a bacterial load that accelerates biodigestion. However, it can also be operated discontinuously, that is, completely filling a container and closing the entry of liquid manure
   until surpassing the hold time (25, 30 days).
- It allows loading the containers in series (complete filling and sequential overflow of the containers) or in parallel (loading and simultaneous partial filling in two or more containers) depending on the liquid manure generation and holding needs (at least 10 days in each container).

The described scalable biodigester plant for generating biogas from livestock liquid manure therefore represents an innovative structure having structural and constitutive features that were unknown up until now for the purpose for which it is intended, and these reasons, along with its practical usefulness, provide sufficient grounds to obtain the exclusive privilege that is sough.

### DESCRIPTION OF THE DRAWINGS

To complement the description that is being made and for the purpose of aiding to better understand the features of the invention, sheets of drawings are attached to the present specification as an integral part thereof, in which the following is depicted in an illustrative and non-limiting manner:
Figure 1 shows a schematic plan view of an embodiment of the scalable biodigester plant, object of the invention, where its general configuration and the main parts comprised therein can be seen.
And Figure 2 shows a schematic section view of an example of the tanks comprised in the plant according to the invention, where the parts and elements comprised therein can be seen.

### PREFERRED EMBODIMENT OF THE INVENTION

In view of the mentioned figures and according to the numbering used, a non-limiting example of the proposed scalable biodigester plant for generating biogas from livestock liquid manure can be seen therein, which plant comprises the parts and elements indicated and described in detail below.

Thus, as can be observed in said figures, the plant (1) in question comprises, in an essential and known manner, a concentration tank (2) with a stirrer (3), to which the liquid manure arrives from the pit of the farm (not shown), which is associated, by means of liquid manure pipe (4), with a pretreatment tank (5) which is associated, in turn, with one or more treatment tanks (6) connected through said liquid manure pipe (4) for the filling thereof.

Furthermore, the plant comprises a heating pipe (7) communicating said tanks (5, 6) with a heater/generator (8) circulating hot water through same to provide the required temperature, a biogas pipe (9) communicating the tanks (5, 6) with the heater/generator (8) for being utilized during the operation thereof, and a discharge pipe (10) communicating the tanks (5, 6) with a solid/liquid separator (11) where the biodigested product is accumulated, separating the solid product (ps) or solid liquid manure (biolsol) and the liquid product (pl) liquid manure (biol) before packaging (e).

The plant (1) differs from this already known configuration in that the tanks (5, 6) are movable structures and consist of standard sized 20' or 40' maritime transport containers, preferably of the open top type, open in the upper part, which incorporate an expandable rubber dome (12), preferably made of EPDM that seals said upper part, allowing only the passage of the biogas pipe (9).

Furthermore, in the preferred embodiment each container making up the tanks (5, 6) is internally lined with a thermally insulating layer (13), preferably made of 30 mm high-density polyurethane that is, in turn, covered with a reinforcement layer (14) made of polyester and glass fiber.

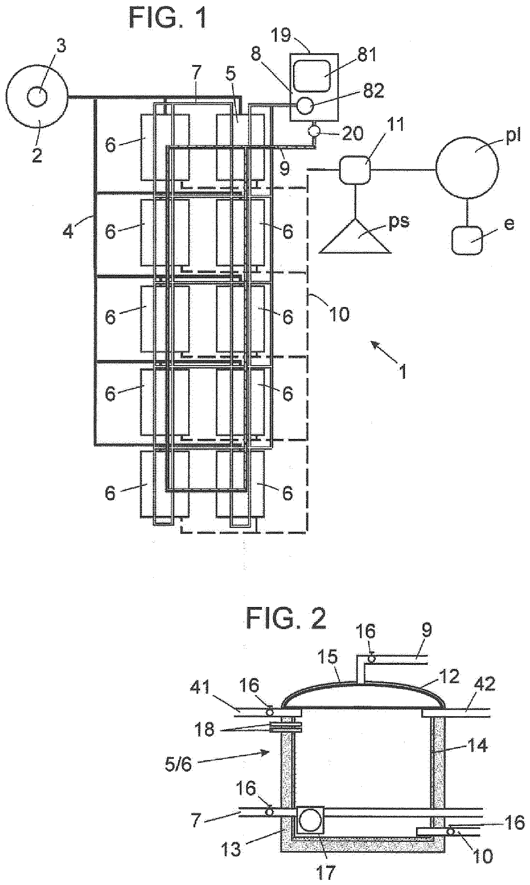

As observed in Figure 2, each tank (5, 6) features an inlet (41) of the liquid manure pipe (4) on one side of the upper part thereof and an outlet (42) at the opposite upper part, being connected such that by means of opening and closing valves (16) interposed in said liquid manure pipe (4), the liquid manure can be loaded in series so that the tanks can be successively filled continuously (filling and overflow of the tank to fill the following tank) or in parallel so that tanks can be filled independently or discontinuously (simultaneously filling two or more containers) depending on the liquid manure generated and the recommendable hold time.

Furthermore, each of said tanks (5, 6) also features the heating pipe (7) in its lower region, the discharge pipe (10) in its lower part and the biogas outlet pipe (9) placed in the upper part of the tank, in the rubber dome (12), which is supported on a structure of rods (15) to maintain a convex curved configuration which prevents the accumulation of rain and/or dust thereon.

Preferably, each liquid manure pipe (4), heating pipe (7), discharge pipe (10), and biogas pipe (9) has in each tank (5, 6) an opening and closing valve (16) which allows the independent use of each tank (5, 6), as appropriate. And likewise, each tank (5, 6) incorporates a pump (17) or another similar device for forcing the liquid manure to circulate through the liquid manure pipe (4), with an activation programmer so it can operate periodically for a few minutes.

Furthermore, each tank also incorporates inlets and outlets for a probe (18) for measuring the temperature or other variables.

Finally, it should be pointed out that the heater/generator (8) comprising a biogas co-generator (81) and a boiler (82) is incorporated in a movable structure (19) which is also a standard maritime transport container. Optionally, the heating pipe (7) coming from said boiler (82) includes a parallel feeder across all the tanks (5, 6) to reduce how far the hot water travels. Furthermore, the incorporation of a sulfuric acid filter (20) in the biogas pipe (9) placed before the inlet thereof into said heater/generator (8) has been envisaged.

Having sufficiently described the nature of the present invention as well as the manner of putting it into practice, it is not considered necessary to extend the description to explain it so that one skilled in the art may comprehend its scope and the advantages derived from it, and it is hereby stated that it may be carried out to practice in other embodiments which differ in detail from the indicated example provided that the essential nature thereof is not modified.

## Claims

1. Scalable biodigester plant for generating biogas from livestock liquid manure, comprising a concentration tank (2) with a stirrer (3), to which the liquid manure arrives from the pit of the farm, associated, by means of liquid manure pipe (4), with a pretreatment tank (5) and one or more treatment tanks (6), there being a heating pipe (7) communicating said tanks (5, 6) with a heater/generator (8) circulating hot water through same to provide the required temperature, a biogas pipe (9) communicating the tanks (5, 6) with the heater/generator (8) for being utilized during the operation thereof, and a discharge pipe (10) communicating the tanks (5, 6) with a solid/liquid separator (11) where the biodigested product is accumulated, **characterized in that** the tanks (5, 6) are movable structures and consist of standard sized 20' or 40' maritime transport containers.

2. Scalable biodigester plant for generating biogas from livestock liquid manure according to claim 1, **characterized in that** the tanks (5, 6) are maritime containers open in the upper part and incorporating an expandable rubber dome (12) that seals said upper part allowing only the passage of the biogas pipe (9).

3. Scalable biodigester plant for generating biogas from livestock liquid manure according to claim 2, **characterized in that** the dome (12) of the tanks (5, 6) is supported on a structure of rods (15).

4. Scalable biodigester plant for generating biogas from livestock liquid manure according to any of claims 1 to 3, **characterized in that** each container making up the tanks (5, 6) is internally lined with a thermally insulating layer (13) that is, in turn, covered with a reinforcement layer (14).

5. Scalable biodigester plant for generating biogas from livestock liquid manure according to claim 4, **characterized in that** the layer of thermal insulation (13) of the tanks (5, 6) is made of 30 mm high-density polyurethane.

6. Scalable biodigester plant for generating biogas from livestock liquid manure according to claim 4 or 5, **characterized in that** the reinforcement layer (14) is made of polyester and glass fiber.

7. Scalable biodigester plant for generating biogas from livestock liquid manure according to any of claims 1 to 6, **characterized in that** each tank (5, 6) features an inlet (41) of the liquid manure pipe (4) on one side of the upper part thereof and an outlet (42) at the opposite upper part.

8. Scalable biodigester plant for generating biogas from livestock liquid manure according to claim 7, **characterized in that** the liquid manure pipe (4) connects the tanks (5, 6) by incorporating opening and closing valves (16) interposed such that the liquid manure can be loaded in series or in parallel, and tanks can be filled independently or discontinuously, depending on the liquid manure generated and the recommendable hold time.

9. Scalable biodigester plant for generating biogas from livestock liquid manure according to any of claims 1 to 8, **characterized in that** each tank (5, 6) features the heating pipe (7) in its lower region, the discharge pipe (10) in its lower part and the biogas outlet pipe (9) placed in the upper part of the tank.

10. Scalable biodigester plant for generating biogas from livestock liquid manure according to any of claims 1 to 9, **characterized in that** each liquid manure pipe (4), heating pipe (7), discharge pipe (10), and biogas pipe (9), has an opening and closing valve (16) which allows the independent use of each tank (5, 6), as appropriate.

11. Scalable biodigester plant for generating biogas from livestock liquid manure according to any of claims 1 to 10, **characterized in that** each tank (5, 6) incorporates a pump (17) or similar device forcing the liquid manure to circulate through the liquid manure pipe (4), with an activation programmer so it can operate periodically for a few minutes.

12. Scalable biodigester plant for generating biogas from livestock liquid manure according to any of claims 1 to 11, **characterized in that** each tank incorporates inlets and outlets for a probe (18) for measuring the temperature or other variables.

13. Scalable biodigester plant for generating biogas from livestock liquid manure according to any of claims 1 to 12, **characterized in that** the heater/generator (8) is incorporated in a movable structure (19) which is also made up of a standard maritime transport container.
